Europäisches Patentamt

(19) European Patent Office

Office européen des brevets

(11) Publication number: **0 149 693**
**B1**

(12) EUROPEAN PATENT SPECIFICATION

(45) Date of publication of patent specification: **29.06.88**

(21) Application number: **84100574.7**

(22) Date of filing: **20.01.84**

(51) Int. Cl.⁴: **A 61 L 27/00, C 08 J 7/04,
C 08 J 7/18**

(54) Method of forming an antithrombogenic layer on medical devices.

(43) Date of publication of application:
**31.07.85 Bulletin 85/31**

(45) Publication of the grant of the patent:
**29.06.88 Bulletin 88/26**

(84) Designated Contracting States:
**BE CH DE FR GB IT LI NL SE**

(56) References cited:
**FR-A-2 142 277
US-A-3 677 800**

**CHEMICAL ABSTRACTS; volume 99, no. 14,
October 3, 1983, page 337, abstract 110707n
(COLUMBUS, OHIO, US) M.F.A. GOOSEN et
al.: "Properties of a heparin-poly(vinyl alcohol)
hydrogel coating", & J. Biomed. Mater. Res.
1983, 17(2), 359-73**

**CHEMICAL ABSTRACTS, volume 99, no. 14,
October 3, 1983, page 338, abstract 110715p
(COLUMBUS, OHIO, US) R. EVANGELISTA et
al.: "Preparation of heparinized biomaterials",
& Proc. IUPAC, Macromolecul. Sympos. 28th,
1982, 357**

(73) Proprietor: **PPG HELLIGE B.V.
I.B.C.-Weg 1
NL-5683 PK Best (NL)**

(72) Inventor: **Kuypers, Martinus Henricus
Schoolstraat 53
NL-5561 AH Riethoven (NL)**

(74) Representative: **Patentanwälte TER MEER -
MÜLLER - STEINMEISTER
Mauerkircherstrasse 45
D-8000 München 80 (DE)**

## Description

The invention relates to a method of forming on medical devices an antithrombogenic layer comprising polyvinyl alcohol and heparin.

Sensor catheter used as an indwelling catheter can monitor physiological values such as pressure, oxygen, pH etc. over some period of time. During a long contact-time, the blood can clot on the surfaces of the sensor-catheter. This clotting is caused by the cascadic biochemical mechanism of the blood, forming deposits called thrombus. This thrombogeneration can be initiated by the bio-incompatibility of a foreign surface in respect to the chemical, physical and mechanical properties of the surface. The thrombus-forming is not only limited to the foreign surface, but the collision of a blood platelet with the foreign surface can irritate the biochemical behaviour of that platelet which can result in thrombus forming in a remote part of the blood circulating system. It is important to control this biochemical reaction to avoid thrombogeneration which can dramatically generate embolism or can at least impair the functionality of the sensor-catheter. In many cases the monitoring with a sensor-catheter will be done during a short time, e.g. during catheterisation for diagnostical purposes. In those cases the thrombogenic processes can be controlled by the administration of anti-coagulantia, of which heparin is the most widely used. However, heparin administration during a longer period of time can lead to adverse effects, e.g. to osteoporosis (softening of the bony tissue) and to the so-called "heparin rebound effect" in which heparin activity returns somewhere in the bloodstream some hours after its neutralisation. During monitoring in the vascular system of the newborn baby, the heparin can initiate even more problems in the instable metabolism of the baby.

As is known from a review article "Heparinised polymers as thromboresistant biomaterials" published by J. E. Wilson in Polym. plast. Technol. Eng 16(2) 119—208 (1981), several trials have been made to bind heparin to the surfaces of medical devices which must be introduced into the bloodstream. In the literature there is still a controversial dispute about the activity and the anti-thrombogenic mechanism of heparin.

According to J. E. Wilson (see above), the antithrombogenity may be caused by the leaching of heparin from the polymer. The micro-atmosphere of the dissolved heparin around the device causes the antithrombogenity. The binding of heparin to the polymer results in a decrease of its activity. Contrary to this explanation M. F. A. Goosen and M. V. Sefton in an article "Properties of a heparin-poly(vinylalcohol)-hydrogel coating" published in the Journal of Biomed. Mat. Res., Vol. 17, 359—373 (1983), showed that chemical binding of heparin in polyvinylalcohol effects positively the antithrombogenity of the polymer. They could prove that no heparin leached out from the P.V.A. hydrogel during their biological activity measurements.

US—A 3 677 800 describes forming a thin continuous adherent coating on a substrate by ultraviolet surface photopolymerization of sodium heparin in the gaseous phase. The resulting coated article exhibits an antithrombogenic behaviour and is suitable for prosthetic use. By photopolymerization a former monomer is converted into a polymer. A similar process is described in US—A 3 625 745 where a synthetic organic antithrombogenic compound is photopolymerized.

It is the main object of the present invention to disclose an improved and economic method of forming antithrombogenic layers on medical devices and in particular on the sensitive surface of medical sensors such as oxygen, pH and pressure sensors. This object is achieved by the new method of forming on medical devices an antithrombogenic layer comprising a hydrogen forming polymer, e.g. polyvinyl alcohol, and heparin, characterized by the steps of:

a) preparing an aqueous solution of a photosensitive hydrogel forming polymer such as polyvinyl alcohol (PVA) or polyvinyl pyrrolidon (PVP), a cross-linking salt and heparin;

b) coating the medical device with said solution;

c) exposing the coated device to ultraviolet radiation.

The new method is easy to perform and in general relies on the well-known principles of lithography. Lithographic masking of parts of a semiconductor or electrically conducting surface is known for generating a fluid-tight cover of those portions to which during a subsequent etching process the etching fluid should not have access. According to the invention this type of process is used for generating an antithrombogenic layer which is permeable at least for that fluid such as an electrolyte or blood which needs to pass into the interior of the sensor for reacting at its electrodes. The method, however, is not only applicable for covering such active surfaces of a sensor but also for coating inactive or passive surface portions of the sensor, a catheter or a medical instrument. A particular advantage of applying this method to sensors is the fact that the same method can be used for depositing other layers and coatings of the sensor such as hydrophylic layers and electrical connections in the form of printed circuits. In this way the same type of manufacturing equipment can be used for depositing different kinds of coatings and layers. The lithographic method and several modifications thereof are described in detail in the book "Photoresist: Materials and Processes" by William S. DeForest, published by McGraw-Hill, Inc. in 1975. In a preferred embodiment of the new method a photosensitive cross-linking agent is used. The invention also extends to medical devices provided with an antithrombogenic coating or layer deposited by performing the method as claimed.

The adhesion of the antithrombogenic layer to a polymeric surface can be achieved by chemical hydrophylising of the polymeric surface by chemical etching in chromic-sulphuric acid, resulting in the formation of C—OH, C=O, —O—C=O

and SO$_3$H groups. The cross-linking agent will also react with some of these groups resulting in an adhering P.V.A.-heparin coating. The heparin is firmly bound to the P.V.A. matrix.

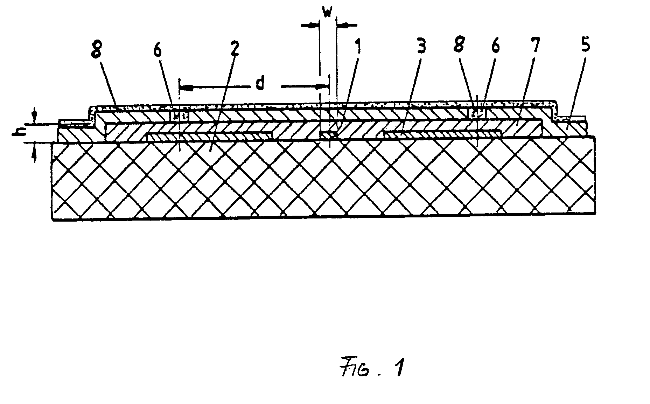

The single drawing shows an oxygen sensor of the Clark type manufactured in accordance with lithographic methods. A silicon substrate 2 supports a cathode 1 and an anode 3 which are deposited on the substrate in the known manner of printed or integrated circuits. Both electrodes are covered by a layer 7 of hydrophylic polymer which is protected on the outside by means of a membrane 5. For activating the sensor either the electrolyte can be deposited as a salt underneath the membrane 5 during the lithographic preparation of the cell, or the electrolyte is derived from the biological fluid surrounding the sensor during its application and then has to be brought into contact with the electrodes and the space between them. For this purpose holes 6 within membrane 5 permit access of the electrolyte to the hydrophylic polymer layer 7 so that the electrolyte can pass along this layer to cathode 1 for activating the cell. Oxygen or any other constituent of the fluid under examination diffuses through membrane 5 and layer 7 to cathode 1, therewith influencing the electrochemical process between electrodes 1 and 3. These electrodes are connected to a source of DC, and the amount of current induced by the reduction and oxidation process at the cathode and the anode, respectively, is a measure for determining the content of oxygen within the biological medium into which the sensor is inserted. Instead of silicon another insulating substrate such as glass or ceramics may be used. When using the cell, it must be prevented that the direct access of oxygen through the holes 6 results in a change or increase of the oxygen diffusion through the selectively permeable membrane 5 to the electrodes. For this reason the distance d of holes 6 from the cathode 1 is by far larger, e.g. five times larger than the width w of the active front surface area of cathode 1. The thickness h of the hydrophylic polymer layer 7 is very small, preferably less than 4 μm. Therewith most or essentially all of the oxygen reaching cathode 1 therefore stems from diffusion through membrane 5 and the oxygen portion entering through holes 6 and travelling along hydrophylic polymer layer 7 can be neglected. This layer may be made of hydrogel such as polyvinyl alcohol, polyvinyl pyrrolidon, polyacrylamide, hydroxyethylmethacrylate or derivates of these compounds. The cathode preferably is made of noble metal such as gold and the anode may consist of silver. The hydrophylic polymer may act as a sieve which is permeable for the electrolyte, oxygen, carbon dioxide and relatively small biological organic molecules such as glucose, but it is impermeable for large biological molecules such as proteins.

In accordance with the present invention an antithrombogenic layer 8 is deposited on the outside surface of membrane 5 covering also the walls of holes 6 and those surface portions of layer 7 which form the bottom of those holes. This layer 8 in accordance with the invention is essentially deposited by the same lithographic method as is used for depositing the hydrophylic layer 7. The hydrophylic-polymer layer 8 containing heparin may be of a structure which is permeable for the electrolyte, oxygen, carbon dioxide and relatively small biological organic molecules such as glucose, but it is impermeable for large biological molecules such as proteins. In this case layer 8 in addition to its antithrombogenic function at least partially provides the sieve function of layer 7.

**Claims**

1. Method of forming on medical devices an antithrombogenic layer comprising a hydrogel forming polymer, e.g. polyvinyl alcohol, and heparin, characterized by the steps of

a) preparing an aqueous solution of photosensitive hydrogen forming polymer such as polyvinyl alcohol (PVA) or polyvinyl pyrrolidon (PVP), a cross-linking agent, and heparin;

b) coating the medical device with said solution;

c) exposing the coated device to ultraviolet radiation.

2. Method according to claim 1 for coating a chemical or physical sensor such as a pressure, pH or oxygen sensor with an antithrombogenic layer, characterized in that the antithrombogenic layer is cross-linked to such an extent that large biological molecules cannot penetrate the interior of the sensor.

3. Method according to claim 1 or 2 of forming an antithrombogenic layer on a polymeric surface, characterized in that before coating the surface with said solution, this surface is chemically hydrophylised by chemical etching in chromic-sulphuric acid.

4. Method according to claim 1, characterized in that the medical device is a catheter.

5. Method according to claim 1, characterized in that the medical device is a medical instrument.

**Patentansprüche**

1. Verfahren zur Bildung eines antithrombogenen Oberflächenüberzuges auf medizinischen Gegenständen mit einem hydrogelbildenden Polymer, zum Beispiel Polyvinylalkohol und Heparin, gekennzeichnet durch folgende Verfahrensschritte:

a) Ansetzen einer wässrigen Lösung aus einem fotoempfindlichen hydrogelbildenden Polymer, wie zum Beispiel Polyvinylalkohol (PVA) oder Polyvinylpyrrolidon (PVP), einem Vernetzungsmittel und Heparin;

b) Beschichten des medizinischen Gegenstandes mit der Lösung;

c) Einwirkenlassen einer ultravioletten Strahlung auf den beschichteten Gegenstand.

2. Verfahren nach Anspruch 1 zur Beschichtung eines chemischen oder physikalischen Sensors für zum Beispiel Druck, pH-Wert oder Sauerstoff mit einem antithrombogenen Oberflächenüberzug, dadurch gekennzeichnet, daß der antithrombogene Oberflächen-überzug in einem solchen Maße vernetzt ist, daß biologische Moleküle nicht in das innere des Sensors eintreten können.

3. Verfahren nach Anspruch 1 oder 2 zur Bildung eines antithrombogenen Oberflächenüberzuges auf einer polymeren Oberfläche, dadurch gekennzeichnet, daß vor Beschichtung der Oberfläche mit der Lösung jene durch chemisches Ätzen in einer Schwefelchromsäure chemisch hydrophyliert wird.

4. Verfahren nach Anspruch 1, dadurch gekennzeichnet, daß der medizinische Gegenstand ein Katheter ist.

5. Verfahren nach Anspruch 1, dadurch gekennzeichnet, daß der medizinische Gegenstand ein medizinisches Instrument ist.

**Revendications**

1. Méthode de formation sur des dispositifs à usage médical d'une couche de produit antithrombogénique comprenant un polymère formant un hydrogel, par exemple l'alcool polyvinylique, et de l'héparine, caractérisée par le fait qu'elle comprend les étapes consistant à:

a) préprer une solution aqueuse d'un polymère photosensible formant un hydrogel tel que l'alcool polyvinylique (PVA) ou la polyvinyl pyrrolidone (PVP), d'un agent de réticulation, et d'héparine;

b) à revêtir ledit dispositif à usage médical avec ladite solution;

c) à exposer le dispositif revêtu à un rayonnement ultraviolet.

2. Méthode selon la revendication 1 pour revêtir un dispositif de détection chimique ou physique tel qu'un détecteur sensible à la pression, au pH ou à l'oxygène, avec une couche de produit antithrombogénique, caractérisée par le fait que la couche antithrombogénique est réticulée de façon que les grosses molécules biologiques ne puissent pénétrar à l'intérieur du capteur.

3. Méthode selon la revendication 1 ou 2, pour former une couche antithrombogénique sur une surface polymérique, caractérisée par le fait qu'avant de revêtir la surface avec ladite solution, cette surface est rendue chimiquement hydrophyle par décapage chimique dans un mélange sulfo-chromique.

4. Méthode selon la revendication 1, caractérisée par le fait que ledit dispositif à usage médical est un cathéter.

5. Méthode selon la revendication 1, caractérisée par le fait que ledit dispositif à usage médical est un instrument médical.

0 149 693

FIG. 1